# EUROPEAN PATENT SPECIFICATION

(11) **EP 2 801 351 B1**
(45) Date of publication and mention of the grant of the patent: **01.08.2018**
(21) Application number: 14167358.2
(22) Date of filing: 07.05.2014
(51) Int. Cl.: A61K 9/50, A61K 9/48, A61K 31/165, A61K 9/20

(54) **MODIFIED RELEASE FORMULATIONS OF LACOSAMIDE**
PHARMAZEUTISCHE FORMULIERUNGEN VON LACOSAMID
FORMULATIONS PHARMACEUTIQUES DE LACOSAMIDE

(30) Priority: 08.05.2013 TR 201305490; 20.06.2013 TR 201307486
(43) Date of publication of application: 12.11.2014
(73) Proprietor: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Kirat Uzunogullari, Nur, 34460 Istanbul (TR); Gülkok, Yildiz, 34460 Istanbul (TR); Saydam, Mehtap, 34460 Istanbul (TR); Eceoglu, Melike, 34460 Istanbul (TR)
(74) Representative: Sevinç, Erkan

(56) References cited:
- EP-A1- 2 468 261
- WO-A1-2011/055385
- WO-A2-2011/101863
- Basf: "Pharma Ingredients & Services Kollicoat MAE grades = Registered trademark of BASF group Methacrylic acid/ethyl acrylate copolymers for enteric coatings", , 1 November 2010 (2010-11-01), XP55329555, Retrieved from the Internet: URL:https://industries.basf.com/bin/bws/do cumentDownload.en.8805243922901 [retrieved on 2016-12-15]

## Description

### Field of Invention

The present invention relates to a modified release formulation comprising lacosamide or a pharmaceutically acceptable salt thereof and an alginate salt or complex salt of alginic acid, together with methacrylic acid-ethyl acrylate copolymer which is in the range of 4-30% by weight of the total formulation.

### Background of Invention

Lacosamide is an aminoacid derivative with an anticonvulsant activity useful in the adjunctive treatment of partial-onset seizures with or without secondary generalization in adults with epilepsy. It is also called as erlosamide or harkoseride. Its chemical name is (2R)-2-(Acetylamino)-3-methoxy-N-(phenylmethyl) propanamide and its chemical structure is shown in the Formula I.

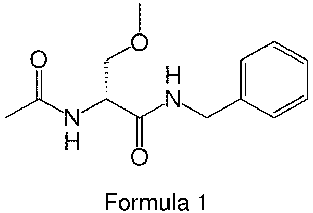

The precise mechanism of antiepileptic effects of lacosamide in humans remains to be fully elucidated. In vitro electrophysiological studies have shown that lacosamide selectively enhances slow inactivation of voltage-gated sodium channels, resulting in stabilization of hyperexcitable neuronal membranes and inhibition of repetitive neuronal firing. Inactivation of sodium channels is important to control of abnormal neuronal activity in the brain of epilepsy patient, and it can occur via fast or slow mechanism. While traditional sodium channel blocking antiepileptics (e.g., Lamotrigine, Carbamazepine, Oxcarbazepine or Phenytoin) act primarly via fast inactivation, lacosamide acts by selectively enhancing slow inactivation of voltage-gated sodium channels to control and stabilize the neural network in the brain.

There is lacosamide immediate release formulation licensed in the US and Europe in the form of immediate release tablets, oral solutions and intravenous injection solutions under the brand name Vimpat® by UCB. Vimpat® IR tablet is prepared by wet granulation comprising crospovidone as disintegrant agent, microcrystalline cellulose, HPC (low substituted) and HPC as filler and binder, silicified microcrystalline cellulose and colloidal silicon dioxide as glidant, magnesium stearate as lubricant and a non-functional coating.

Although there is no modified release formulation of lacosamide available in the market, immediate release tablets are currently commercially available in strengths of 50, 100, 150 and 200 mg of lacosamide. It may be taken with or without food. The initial dose should be 50 mg twice daily (100 mg per day) and it can be increased at weekly intervals by 100 mg/day given as two divided doses up to the recommended maintenance dose of 200 to 400 mg/day, based on individual patient response and tolerability.

Pharmacokinetics of lacosamide are dose proportional (100-800 mg) and time invariant, with low inter- and intra-subject variability. Following oral administration of lacosamide, the plasma concentration increases rapidly and reaches Cₘₐₓ about 0.5 to 4 hours. It leads twice daily dosing of immediate release lacosamide tablet. Multiple daily dosing leads to fluctuations on concentration of lacosamide in the blood stream and a steady plasma concentration can be achieved in a three day period or more. These pharmacokinetic properties cause serious side effects which decrease the patient's quality of life. The most frequent side effects are dizziness, headache, nausea and diplopia, in decreasing order and side effects prevent the increase in the dose of lacosamide. In this invention, pharmaceutically acceptable dosage form has been specifically formulated as to contain lacosamide and release slowly it over in a desired period of time, until the next dosage is administered. In that way, side effects caused by high dose of lacosamide has been prevented and an effective and safe administration has been achieved.

Furthermore, lacosamide is sparingly soluble in water and it has flowability problem which makes the tableting process difficult at the high amount of active substance. It is very crucial to choose suitable excipients and process to overcome compressibility problem and achieve desired dissolution profile at the same time. In this invention, alginic acid salts have been used with methacrylic acid-ethyl acrylate copolymer to obtain modified release tablets.

WO2011/055385 (A1) relates to modified release lacosamide formulations. Modified release polymers such as hydrophilic polymer, hydrophobic polymer, wax are employed in lacosamide formulations. WO2011/055385 (A1) addressed the need for modified release formulation of lacosamide which controls the release of lacosamide in such a manner that therapeutically effective concentration is maintained in the blood for an extended period of time keeping the drug concentration in the blood substantially constant. Flowability and compressibility issues in modified release lacosamide formulations are not mentioned within WO2011/055385 (A1). Further, WO2011/055385 (A1) is also silent on the use of methacrylic acid-ethyl acrylate copolymer in the range of 4%-30% by weight of the total formulation.

EP 2 468 261 (A1) is directed to controlled release formulations of lacosamide for oral administration. EP 2 468 261 (A1) is mainly focused on the need for lacosamide formulations having decreased side effect/efficacy ratio and benefit/risk ratio. EP 2 468 261 (A1) does not propose any solution for eliminating the risk of dose-dumping in modified release formulations as well as for improving flowability and compressibility characteristics. The specific ratio of methacrylic acid-ethyl acrylate copolymer is absent in EP 2 468 261 (A1).

WO 2011/101863 (A2) relates to extended release pharmaceutical compositions of lacosamide. Even the release profile is known from this prior art document, there is no mention to lacosamide formulations comprising an alginate salt or complex salt of alginic acid together with methacrylic acid-ethyl acrylate copolymer in the range of 4%-30% by weight of the total formulation. Further, flowability, compressibility and dose-dumping problems are not mentioned throughout the teaching of WO 2011/101863 (A2).

Accordingly, there is a need for an improved formulation of lacosamide or pharmaceutically acceptable salt to overcome the above described problems, minimize the adverse effects and provide once daily usage with an optimum drug plasma concentration which is equivalent or better than the immediate release tablet.

### Description of the invention

The main embodiment of the present invention is to provide a modified release formulation comprising lacosamide or a pharmaceutically acceptable salt thereof and an alginate salt or complex salt of alginic acid, together with methacrylic acid-ethyl acrylate copolymer; wherein methacrylic acid-ethyl acrylate copolymer is in the range of 4-30% by weight of the total formulation.

According to one embodiment of the present invention is to obtain a modified release formulation comprising lacosamide for once daily usage with or without food.

According to another object, lacosamide or a pharmaceutically acceptable salt thereof is present in an amount of about 5 to 80 % by weight of total formulation; preferably it is about 20 to 60 % by weight of total formulation.

Alginate salts and complex salts of alginic acids are hydrocolloids, water-soluble biopolymers. In pharmaceutical formulations, they can be used as a binder or a disintegrant. Moreover, they can delay the dissolution of a drug by forming gel matrices. In this invention, alginic acids have shown better effects on both disintegration and dissolution on lacosamide modified release formulation. Improved dissolution of lacosamide has been achieved. Additionally, flowability problem of lacosamide could also be overcome and compressibility has been enhanced due to viscosity and binding property of alginate salts and complex salts of alginic acids.

According to one embodiment, the complex salt of alginic acid is sodium-calcium alginate with the range of 0.1 - 25 % by weight of total formulation, preferably it is 2 - 20 % by weight of total formulation.

The term "alginate salt" includes substance such as calcium alginate, potassium alginate, sodium alginate, propylene glycol alginate, ammonium alginate or mixtures thereof.

In one embodiment, the alginate salt is sodium alginate with the range of 0.1 - 25 % by weight of total formulation, preferably it is 2 - 20 % by weight of total formulation.

As used herein, the term "matrix retarding agent" is defined as the pharmaceutical ingredient that provides modified release, retarded release, slow release, controlled release, sustained release, prolonged release, zero point order release or extended release.

The pharmaceutically acceptable matrix retarding agent is methacrylic acid - ethyl acrylate copolymer (Eudragit L100-55, Kollicoat MAE 30DP).

The matrix retarding agent, which is methacrylic acid - ethyl acrylate copolymer (Eudragit L100-55, Kollicoat MAE 30DP) is used in the range of 4 - 30 % by weight of total formulation.

Methacrylic acid - Ethyl acrylate copolymers are film forming agents for a preferred, convenient and cost-saving enteric coatings of solid oral dosage forms in the state of art.

They contain an anionic copolymer based on methacrylic acid and ethyl acrylate. They are effective and stable enteric coatings with a desired dissolution for modified release. In this invention, to enhance the effect of alginate salt or complex salt of alginic acid and obtain desired optimum dissolution, methacrylic acid - ethyl acrylate copolymers have been used in modified release formulation of lacosamide.

According to one embodiment, the modified release formulation comprises the alginate salt is selected from the group comprising calcium alginate, potassium alginate, sodium alginate, propylene glycol alginate, ammonium alginate or mixtures. , and methacrylic acid - ethyl acrylate copolymer as a matrix retarding agent.

According to the present invention, the modified release formulation comprises an alginate salt or complex salt of alginic acid and methacrylic acid - ethyl acrylate copolymer.

According to another embodiment, the formulation releases 8 to 40 % of the total amount of lacosamide or a pharmaceutically acceptable salt thereof in 1 hour, 20 to 75 % in 2 hours, 40 to 80 % in 4 hours and minimum 85% in 8 hours, when in- vitro release of lacosamide is measured according to USP (edition 24) method <711>, in 900mL of 0.1N HCl at 75 rpm.

In this present invention, to achieve modified release and improve tablet compressibility, these formulations have been designed, comprising the following:
a. 5.0 - 80.0 % by weight of lacosamide
b. 5.0 - 40.0 % by weight of dibasic calcium phosphate
c. 0.1 - 25.0 % by weight of sodium alginate
d. 4.0 - 30.0 % by weight of methacrylic acid - ethyl acrylate copolymers
e. 0.1 - 0.2 % by weight of talc
f. 0.25 - 2.0 % by weight of sodium stearyl fumarate,

a. 5.0 - 80.0 % by weight of lacosamide
b. 5.0 - 40.0 % by weight of dibasic calcium phosphate
c. 0.1 - 25.0 % by weight of sodium - calcium alginate complex
d. 4.0 - 30.0 % by weight of methacrylic acid - ethyl acrylate copolymers
e. 0.1 - 0.2 % by weight of talc
f. 0.25 - 2.0 % by weight of sodium stearyl fumarate

a. 5.0 - 80.0 % by weight of lacosamide
b. 0.1 - 25.0 % by weight of sodium alginate
c. 4.0 - 30.0 % by weight of methacrylic acid - ethyl acrylate copolymers
d. 0.1 - 0.2 % by weight of talc
e. 0.25 - 2.0 % by weight of sodium stearyl fumarate

a. 5.0 - 80.0 % by weight of lacosamide
b. 0.1 - 25.0 % by weight of sodium - calcium alginate complex
c. 4.0 - 30.0 % by weight of methacrylic acid - ethyl acrylate copolymers
d. 0.1 - 0.2 % by weight of talc
e. 0.25 - 2.0 % by weight of sodium stearyl fumarate,

a. 5.0 - 80.00 % by weight of lacosamide
b. 0.1 - 25 % by weight of sodium alginate
c. 4.0 - 30 % by weight of methacrylic acid - ethyl acrylate copolymers
d. 1.0 - 40 % by weight of diethyl phthalate
e. 0.5 - 40 % by weight polyvinyl acetate
f. 0.1 - 0.2 % by weight of talc
g. 0.25 - 2.0 % by weight of sodium stearyl fumarate
h. 5.0 - 90.0 % sugar pellet
and
a. 5.0 - 80.00 % by weight of lacosamide
b. 0.1 - 25 % by weight of sodium - calcium alginate complex
c. 4.0 - 30.0 % by weight of methacrylic acid - ethyl acrylate copolymers
d. 1.0 - 40 % by weight of diethyl phthalate
e. 0.5 - 40 % by weight polyvinyl acetate
f. 0.1 - 0.2 % by weight of talc
g. 0.25 - 2.0 % by weight of sodium stearyl fumarate
h. 5.0 - 90.0 % sugar pellet

The modified release formulations of this invention further comprising at least one pharmaceutically acceptable excipient selected from the group comprising diluents, fillers, binders, lubricants, glidants and preferably a moisture protective coating.

Suitable diluents and fillers may include but not limited to dibasic calcium phosphate, tribasic calcium phosphate, trehalose, isomalt, microcrystalline cellulose, mannitol, lactose, starch, sodium carbonate, sodium bicarbonate, calcium carbonate or mixtures thereof, preferably dibasic calcium phosphate.

Another advantage of this invention is to comprise dibasic calcium phosphate. It is insoluble and non-elastic diluent and filler provides a resistance which is required in the tablet matrix comprising a swelling agent. In this invention, to further enhance the compressibility of lacosamide, dibasic calcium phosphate is used due to its compaction and flowability properties in wet granulation.

Suitable lubricants may include but not limited to sodium stearyl fumarate, stearic acid, magnesium strearate, calcium stearate or mixtures thereof; preferably sodium stearyl fumarate.

Suitable glidants may include but not limited to colloidal silicon dioxide, talc, aluminium silicate and the like or mixtures thereof; preferably talc.

Coating may also preferably be used for moisture protection. It can be selected from the group comprising Polyvinyl alcohol-polyethylene glycol copolymers (Kollicoat IR), polyvinyl alcohol or copolymers or mixtures thereof (Opadry AMB, OPADRY 200), Ethylcellulose Dispersions (Surelease), Kerry-HPC, polyethylene glycol, polyvinylprolidone, polyvinylprolidone-vinyl acetate copolymer(PVP-VA), and all kinds of OpadryTM, as well as pigments, dyes, titanium dioxide and iron oxide, talk and polymethylmetacrylate copolymers (Eudragit).

### Example 1: (Extrusion/Spheronisation, Core Pelletizasyon)

| **ingredients** | **amount (%)** |
|---|---|
| Lacosamide | 20.0 - 60.0 |
| dibasic calcium phosphate | 5.0 - 40.0 |
| Sodium alginate | 2 - 20.0 |
| methacrylic acid - ethyl acrylate copolymers | 4 - 30.0 |
| talc | 0.1 - 0.2 |
| sodium stearyl fumarate | 0.25 - 2.0 |

The process of the formulation is carried out as follows: Lacosamide, dibasic calcium phosphate and sodium alginate are mixed and granulated with hidroalcoholic/alcoholic sodium alginate solution. Granules are passed through the extruder and pellets are obtained by spheronization. Pellets are coated with the solution of methacrylic acid - ethyl acrylate copolymer. Coated pellets are mixed with talc and sodium stearyl fumarate respectively and pressed into tablets or filled into capsules. Pressed tablets are preferably coated with Opadry AMB /Opadry 200 or Kollicoat IR.

### Example 2: (spray drying)

| **ingredients** | **amount (%)** |
|---|---|
| Lacosamide | 20.0 - 60.0 |
| Sodium alginate | 2 - 20.0 |
| methacrylic acid - ethyl acrylate copolymers | 4 - 30.0 |
| talc | 0.1 - 0.2 |
| sodium stearyl fumarate | 0.25 - 2.0 |

The process of the formulation is carried out as follows: hydroalcoholic/alcoholic solution is prepared by mixing of lacosamide and sodium alginate. Pellets are obtained in spray drier. Pellets are coated with solution of methacrylic acid - ethyl acrylate copolymers. Coated pellets are mixed with talc and sodium stearyl fumarate respectively and pressed into tablets or filled into capsules. Pressed tablets are preferably coated with Opadry AMB /Opadry 200 or Kollicoat IR.

### Example 3: (sugar pellet coating)

| **ingredients** | **amount (%)** |
|---|---|
| Lacosamide | 20.0 - 60.0 |
| Sodium alginate | 2 - 20.0 |
| methacrylic acid - ethyl acrylate copolymers | 4 - 30.0 |
| diethyl phthalate | 1.0 - 40.0 |
| polyvinyl acetate (Kollidon SR) | 0.5 - 40.0 |
| talc | 0.1 - 0.2 |
| sodium stearyl fumarate | 0.25 - 2.0 |
| sugar pellet | 5.0 - 90.0 |

The process of the formulation is carried out as follows: hydroalcoholic/alcoholic solution/dispersion prepared by mixing lacosamide and sodium alginate . Then, sugar pellets are coated with this solution/dispersion. Then, active agent coated sugar pellets are coated with methacrylic acid - ethyl acrylate copolymers, diethyl phthalate solution and polyvinyl acetate respectively. Coated pellets are mixed with talc and sodium stearyl fumarate respectively and pressed into tablets or filled into capsules. Pressed tablets are preferably coated with Opadry AMB /Opadry 200 or Kollicoat IR.

### Example 4: (Extrusion/Spheronisation, Core Pelletizasyon)

| **ingredients** | **amount (%)** |
|---|---|
| Lacosamide | 20.0 - 60.0 |
| dibasic calcium phosphate | 5.0 - 40.0 |
| sodium - calcium alginate complex | 2 - 20.0 |
| methacrylic acid - ethyl acrylate copolymers | 4 - 30.0 |
| talc | 0.1 - 0.2 |
| sodium stearyl fumarate | 0.25 - 2.0 |

The process of the formulation is carried out as follows: Lacosamide, dibasic calcium phosphate and sodium - calcium alginate complex are mixed and granulated with hidroalcoholic/alcoholic sodium alginate solution. Granules are passed through the extruder and pellets are obtained by spheronization. Pellets are coated with the solution of methacrylic acid - ethyl acrylate copolymer. Coated pellets are first mixed with talc and sodium stearyl fumarate respectively and pressed into tablets or filled into capsules. Pressed tablets are preferably coated with Opadry AMB /Opadry 200 or Kollicoat IR.

### Example 5: (spray drying)

| **ingredients** | **amount (%)** |
|---|---|
| Lacosamide | 20.0 - 60.0 |
| sodium - calcium alginate complex | 2 - 20.0 |
| methacrylic acid - ethyl acrylate copolymers | 4 - 30.0 |
| talc | 0.1 - 0.2 |
| sodium stearyl fumarate | 0.25 - 2.0 |

The process of the formulation is carried out as follows: hydroalcoholic/alcoholic solution is prepared by mixing of lacosamide and sodium - calcium alginate complex. Pellets are obtained in spray drier. Pellets are coated with solution of methacrylic acid - ethyl acrylate copolymers. Coated pellets are mixed with talc and sodium stearyl fumarate respectively and pressed into tablets or filled into capsules. Pressed tablets are preferably coated with Opadry AMB /Opadry 200 or Kollicoat IR.

### Example 6: (sugar pellet coating)

| **ingredients** | **amount (%)** |
|---|---|
| Lacosamide | 20.0 - 60.0 |
| sodium - calcium alginate complex | 2 - 20.0 |
| methacrylic acid - ethyl acrylate copolymers | 4 - 30.0 |
| diethyl phthalate | 1.0 - 40.0 |
| polyvinyl acetate (Kollidon SR) | 0.5 - 40.0 |
| talc | 0.1 - 0.2 |
| sodium stearyl fumarate | 0.25 - 2.0 |
| sugar pellet | 5.0 - 90.0 |

The process of the formulation is carried out as follows: hydroalcoholic/alcoholic solution/dispersion prepared by mixing of process lacosamide and sodium - calcium alginate complex and sugar pellets are coated with this solution/dispersion. Then, active agent coated sugar pellets are coated with methacrylic acid - ethyl acrylate copolymers, diethyl phthalate solution and polyvinyl acetate respectively. Coated pellets are mixed with talc and sodium stearyl fumarate respectively and pressed into tablets. Pressed tablets are preferably coated with Opadry AMB /Opadry 200 or Kollicoat IR.

## Claims

1. A modified release formulation comprising lacosamide or a pharmaceutically acceptable salt thereof and an alginate salt or complex salt of alginic acid together with methacrylic acid -ethyl acrylate copolymer; wherein methacrylic acid-ethyl acrylate copolymer is in the range of 4-30% by weight of the total formulation.

2. The modified release formulation according to any of the preceding claims comprising;
a) 5.00 - 80.00 % by weight of lacosamide
b) 5.00 - 40.00 % by weight of dibasic calcium phosphate
c) 0.10 - 25.00 % by weight of sodium alginate
d) 4.00 - 30.00 % by weight of methacrylic acid - ethyl acrylate copolymers
e) 0.10 - 0.20 % by weight of talc
f) 0.25 - 2.00 % by weight of sodium stearyl fumarate

3. The modified release formulation according to any of the preceding claims comprising;
a) 5.0 - 80.0 % by weight of lacosamide
b) 5.0 - 40.0 % by weight of dibasic calcium phosphate
c) 0.1 - 25.0 % by weight of sodium - calcium alginate complex
d) 4.0 - 30.0% by weight of methacrylic acid - ethyl acrylate copolymers
e) 0.1 - 0.2 % by weight of talc
f) 0.25 - 2.0 % by weight of sodium stearyl fumarate

4. The modified release formulation according to any of the preceding claims comprising;
a) 5.00 - 80.00 % by weight of lacosamide
b) 0.10 - 25.00 % by weight of sodium alginate
c) 4.00 - 30.00 % by weight of methacrylic acid - ethyl acrylate copolymers
d) 0.10 - 0.20 % by weight of talc
e) 0.25 - 2.00 % by weight of sodium stearyl fumarate

5. The modified release formulation according to any of the preceding claims comprising;
a) 5.0 - 80.0 % by weight of lacosamide
b) 0.1 - 25.0 % by weight of sodium - calcium alginate complex
c) 4.0 - 30.0 % by weight of methacrylic acid - ethyl acrylate copolymers
d) 0.1 - 0.2 % by weight of talc
e) 0.25 - 2.0 % by weight of sodium stearyl fumarate

6. The modified release formulation according to any of the preceding claims comprising;
a) 5.0 - 80.0 % by weight of lacosamide
b) 0.1 - 25.0 % by weight of sodium alginate
c) 4.0 - 30.0 % by weight of methacrylic acid - ethyl acrylate copolymers
d) 1.0 - 40.0 % by weight of diethyl phthalate
e) 0.5 - 40.0 % by weight polyvinyl acetate
f) 0.1 - 0.2 % by weight of talc
g) 0.25 - 2.0 % by weight of sodium stearyl fumarate
h) 5.0 - 90.0 % sugar pellet

7. The modified release formulation according to any of the preceding claims comprising;
a) 5.0 - 80.0 % by weight of lacosamide
b) 0.1 - 25.0 % by weight of sodium - calcium alginate complex
c) 4.0 - 30.0% by weight of methacrylic acid - ethyl acrylate copolymers
d) 1.0 - 40.0 % by weight of diethyl phthalate
e) 0.5 - 40.0 % by weight polyvinyl acetate
f) 0.1 - 0.2 % by weight of talc
g) 0.25 - 2.0 % by weight of sodium stearyl fumarate
h) 5.0 - 90.0 % sugar pellet

## Patentansprüche

1. Modifizierter Freigabeansatz umfassend Lacosamid oder ein pharmazeutisch akzeptables Salz davon und ein Alginatsalz oder ein komplexes Salz von Alginsäure zusammen mit Methacrylsäure-Ethylacrylatcopolymer; wobei Methacrylsäure Ethylacrylatcopolymer im Bereich von 4-30 Gew.-% des Gesamtansatzes vorhanden ist.

2. Modifizierter Freigabeansatz nach irgendeinem der vorhergehenden Ansprüche mit:
a) 5,00 - 80,00 Gew.-% Lacosamid
b) 5,00 - 40,00 Gew.-% dibasischem Calciumphosphat
c) 0,10 - 25,00 Gew.-% Natriumalginat
d) 4,00 - 30,00 Gew.-% Methacrylsäure-Ethylacrylatcopolymeren
e) 0,10 - 0,20 Gew.-% Talk
f) 0,25 - 2,00 Gew.-% Natriumstearylfumarat.

3. Modifizierter Freigabeansatz nach irgendeinem der vorhergehenden Ansprüche mit:
a) 5,00 - 80,00 Gew.-% Lacosamid
b) 5,00 - 40,00 Gew.-% dibasischem Calciumphosphat
c) 0,1 - 25,00 Gew.-% Natrium-Calcium-Alginatkomplex
d) 4,0 - 30,00 Gew.-% Methacrylsäure-Ethylacrylatcopolymeren
e) 0,1 - 0,2 Gew.-% Talk
f) 0,25 - 2,0 Gew.-% Natriumstearylfumarat.

4. Modifizierter Freigabeansatz nach irgendeinem der vorhergehenden Ansprüche mit:
a) 5,00 - 80,00 Gew.-% Lacosamid
b) 0,10 - 25,00 Gew.-% Natriumalginat
c) 4,00 - 30,00 Gew.-% Methacrylsäure-Ethylacrylatcopolymeren
d) 0,10 - 0,20 Gew.-% Talk
e) 0,25 - 2,00 Gew.-% Natriumstearylfumarat.

5. Modifizierter Freigabeansatz nach irgendeinem der vorhergehenden Ansprüche mit:
a) 5,00 - 80,00 Gew.-% Lacosamid
b) 0,1 - 25,00 Gew.-% Natrium-Calcium-Alginatkomplex
c) 4,0 - 30,00 Gew.-% Methac- rylsäure-Ethylacrylatcopolymeren
d) 0,1 - 0,2 Gew.-% Talk
e) 0,25 - 2,0 Gew.-% Natriumstearylfumarat.

6. Modifizierter Freigabeansatz nach irgendeinem der vorhergehenden Ansprüche mit:
a) 5,00 - 80,0 Gew.-% Lacosamid
b) 0,1 - 25,0 Gew.-% Natriumalginat
c) 4,0 - 30,0 Gew.-% Methacrylsäure-Ethylacrylatcopolymeren
d) 1,0 - 40,0 Gew.-% Diethylphthalat
e) 0,5 - 40,0 Gew.-% Polyvinylacetat
f) 0,1 - 0,2 Gew.-% Talk
g) 0,25 - 2,0 Gew.-% Natriumstearylfumarat
h) 5,0 - 90,0 Gew.-% Zuckerpellets.

7. Modifizierter Freigabeansatz nach irgendeinem der vorhergehenden Ansprüche mit:
a) 5,0 - 80,00 Gew.-% Lacosamid
b) 0,1 - 25,0 Gew.-% Natrium-Calcium-Alginatkomplex
c) 4,0 - 30,0 Gew.-% Methacrylsäure-Ethylacrylatcopolymeren
d) 1,0 - 40,0 Gew.-% Diethylphthalat
e) 0,5 - 40,0 Gew.-% Polyvinylacetat
f) 0,1 - 0,2 Gew.-% Talk
g) 0,25 - 2,0 Gew.-% Natriumstearylfumarat
h) 5,0 - 90,0 Gew.-& Zuckerpellets.

## Revendications

1. Formulation à libération modifiée comprenant du lacosamide ou un sel pharmaceutiquement acceptable de celui-ci et un sel alginate ou sel complexe d'acide alginique conjointement avec un copolymère acide méthacrylique-acrylate d'éthyle, dans laquelle le copolymère acide méthacrylique-acrylate d'éthyle se situe dans la plage de 4-30 % en poids de la formulation totale.

2. Formulation à libération modifiée selon l'une quelconque des revendications précédentes, comprenant :
a) 5,00-80,00 % en poids de lacosamide ;
b) 5,00-40,00 % en poids de phosphate de calcium dibasique ;
c) 0,10-25,00 % en poids d'alginate de sodium ;
d) 4,00-30,00 % en poids de copolymères acide méthacrylique-acrylate d'éthyle ;
e) 0,10-0,20 % en poids de talc ;
f) 0,25-2,00 % en poids de fumarate de sodium et de stéaryle.

3. Formulation à libération modifiée selon l'une quelconque des revendications précédentes, comprenant :
a) 5,0-80,0 % en poids de lacosamide ;
b) 5,0-40,0 % en poids de phosphate de calcium dibasique ;
c) 0,1-25,0 % en poids de complexe d'alginate de sodium - calcium ;
d) 4,0-30,0 % en poids de copolymères acide méthacrylique-acrylate d'éthyle ;
e) 0,1-0,2 % en poids de talc ;
f) 0,25-2,0 % en poids de fumarate de sodium et de stéaryle.

4. Formulation à libération modifiée selon l'une quelconque des revendications précédentes, comprenant :
a) 5,00-80,00 % en poids de lacosamide ;
b) 0,10-25,00 % en poids d'alginate de sodium ;
c) 4,00-30,00 % en poids de copolymères acide méthacrylique-acrylate d'éthyle ;
d) 0,10-0,20 % en poids de talc ;
e) 0,25-2,00 % en poids de fumarate de sodium et de stéaryle.

5. Formulation à libération modifiée selon l'une quelconque des revendications précédentes, comprenant :
a) 5,0-80,0 % en poids de lacosamide ;
b) 0,1-25,0 % en poids d'un complexe d'alginate de sodium - calcium ;
c) 4,0-30,0 % en poids de copolymères acide méthacrylique-acrylate d'éthyle ;
d) 0,1-0,2 % en poids de talc ;
e) 0,25-2,0 % en poids de fumarate de sodium et de stéaryle.

6. Formulation à libération modifiée selon l'une quelconque des revendications précédentes, comprenant :
a) 5,0-80,0 % en poids de lacosamide ;
b) 0,1-25,0 % en poids d'alginate de sodium ;
c) 4,0-30,0 % en poids de copolymères acide méthacrylique-acrylate d'éthyle ;
d) 1,0-40,0 % en poids de phtalate de diéthyle ;
e) 0,5-40,0 % en poids de poly(acétate de vinyle) ;
f) 0,1-0,2 % en poids de talc ;
g) 0,25-2,0 % en poids de fumarate de sodium et de stéaryle ;
h) 5,0-90,0 % de granules de sucre.

7. Formulation à libération modifiée selon l'une quelconque des revendications précédentes, comprenant :
a) 5,0-80,0 % en poids de lacosamide ;
b) 0,1-25,0 % en poids de complexe d'alginate de sodium-calcium ;
c) 4,0-30,0% en poids de copolymères acide méthacrylique-acrylate d'éthyle ;
d) 1,0-40,0 % en poids de phtalate de diéthyle ;
e) 0,5-40,0 % en poids de poly(acétate de vinyle) ;
f) 0,1-0,2 % en poids de talc ;
g) 0,25-2,0 % en poids de fumarate de sodium et de stéaryle ;
h) 5,0-90,0 % de granules de sucre.
